# EUROPÄISCHE PATENTSCHRIFT

(11) **EP 3 284 498 B1**
(45) Veröffentlichungstag und Bekanntmachung des Hinweises auf die Patenterteilung: **24.07.2019**
(21) Anmeldenummer: 17001491.4
(22) Anmeldetag: 05.02.2014
(51) Int. Cl.: A61M 5/315, A61M 5/24

(54) **INJEKTIONSGERÄT**
INJECTION DEVICE
APPAREIL D'INJECTION

(30) Priorität: 08.02.2013 DE 202013001350 U
(43) Veröffentlichungstag der Anmeldung: 21.02.2018
(62) Teilanmeldung aus: 14703269.2
(73) Patentinhaber: Haselmeier AG, 9001 St. Gallen (CH)
(72) Erfinder: Keitel, Joachim, 73728 Esslingen (DE); MacDonald, Daniel, Brossard, Québec J4Z 0J4 (CA); Bechtold, Herbert, 78588 Denkingen (DE)
(74) Vertreter: Reinhardt, Annette

(56) Entgegenhaltungen:
- EP-B2- 1 610 848
- WO-A1-2006/076921
- WO-A1-2010/140974
- WO-A1-2011/101349

## Beschreibung

Die Erfindung betrifft ein Injektionsgerät der im Oberbegriff des Anspruchs 1 angegebenen Gattung.

Aus der EP 1 610 848 B2 ist ein Injektionsgerät bekannt, bei dem zum Einstellen einer Injektionsdosis ein Dosierorgan gedreht wird, bis die gewünschte Dosis in einem Sichtfenster erscheint. Aufgrund der Drehung bewegt sich das Dosierorgan in distaler Richtung des Injektionsgeräts, entfernt sich also von einer auf das Injektionsgerät aufgesetzten Injektionsnadel. Das Injektionsgerät besitzt eine Rasteinrichtung, die eine Vielzahl von Raststellungen besitzt. Der Bediener kann die Dosis dadurch auch durch Abzählen einer Anzahl von spürbaren und hörbaren Raststellungen einstellen. Ein Einstellen von Zwischenstellungen zwischen zwei Raststellungen ist nicht möglich. Das Injektionsgerät kann eine Torsionsfeder besitzen, die beim Einstellen einer Dosis funktionslos ist. Beim Auspressen einer eingestellten Dosis aus dem Behälter unterstützt die Torsionsfeder die Verdrehung des Einstellglieds und damit die Injektion.

Bei dem in der EP 1 610 848 B2 gezeigten Injektionsgerät können unterschiedlichste Mengen an Injektionsflüssigkeit eingestellt werden. Die möglichen einzustellenden Mengen werden über die Rasteinrichtung vorgegeben. Von Positionen des Bedienelements, die vom Hersteller nicht vorgesehenen Mengen an Injektionsflüssigkeit entsprechen, springt das Bedienelement in die nächstliegende vorgesehene Position.

Damit der Bedienknopf aus einer Zwischenposition selbsttätig und sicher in eine Rastposition springt, muss die Raste hinreichend stark sein, und die radialen Rastpositionen müssen hinreichend nahe beieinander liegen. Die Stärke der Rastung beeinflusst jedoch das Drehmoment, das der Anwender aufwenden muss, um den Bedienknopf zu drehen und die Dosis einzustellen. Der konstruktiv mögliche Abstand der Rastpositionen ist dadurch weitgehend vorgegeben und kann nur in engen Grenzen auf den Anwendungsfall angepasst werden.

Aus der WO 2011/101349 A1 geht ein Injektionsgerät mit fest eingestellter Dosis hervor. Wenn der Bediener den Bedienknopf nicht um einen ausreichenden Winkel dreht, um eine Raststellung zu erreichen, wird keine Dosis eingestellt, sondern der Dosiseinstellknopf wird zurück in die Nullstellung gedreht.

Die WO 2006/076921 A1 zeigt ein automatisches Injektionsgerät mit einer Torsionsfeder. Es ist eine Rasteinrichtung vorgesehen, die verhindert, dass das Dosiseinstellelement sich aufgrund der Kraft der Torsionsfeder zurück in seine Ausgangsstellung drehen kann.

Die WO 2010/140974 A1 zeigt ein Injektionsgerät, dessen Skalenrohr über eine Gewindeverbindung mit einem Antriebselement verbunden ist. Das Antriebselement besitzt eine Rastverbindung, die so ausgebildet ist, dass das Antriebselement nur in eine Richtung gegenüber der Antriebsmutter gedreht werden kann.

Der Erfindung liegt die Aufgabe zugrunde, ein Injektionsgerät der gattungsgemäßen Art zu schaffen, bei dem nur vorgegebene Mengen von Injektionsflüssigkeit aus dem Behälter ausgepresst werden können und das eine gute Anpassung auf den gewünschten Anwendungsfall erlaubt.

Diese Aufgabe wird durch ein Injektionsgerät mit den Merkmalen des Anspruchs 1 gelöst.

Das Dosierorgan besitzt mindestens eine Injektionsstellung, in der eine vom Hersteller vorgesehene Dosis an Injektionsflüssigkeit eingestellt ist. Dabei kann nur eine einzige Injektionsstellung vorgesehen sein, wenn ein Medikament zu verabreichen ist, das nur in einer vorgegebenen Dosis verabreicht werden soll. Es können jedoch auch mehrere Injektionsstellungen vorgesehen sein, beispielsweise, wenn ein Medikament abhängig von Indikation und Bediener in mehreren unterschiedlichen vorgegebenen Dosen verabreicht werden kann. Das Injektionsgerät besitzt eine Rasteinrichtung, wobei jeder Injektionsstellung des Dosierorgans eine Raststellung der Rasteinrichtung zugeordnet ist. Je nach Auslegung der Rasteinrichtung kann die von der Rasteinrichtung auf das Dosierorgan ausgeübte Kraft zu klein sein, um das Dosierorgan aus jeder Zwischenstellung zwischen der Nullstellung und der Injektionsstellung bzw. aus jeder Zwischenstellung zwischen zwei Injektionsstellungen selbsttätig und sicher in die Nullstellung oder eine Injektionsstellung zurückzustellen. Um bei einem Injektionsgerät, bei dem das Dosierorgan aufgrund der Auslegung der Rasteinrichtung in eine Zwischenstellung gestellt werden kann, zu vermeiden, dass ein Bediener die nicht vorgesehene Dosis, also eine Dosis, die einer Zwischenstellung entspricht, injizieren kann, wirkt zwischen dem Dosierorgan und dem Gehäuse eine Feder. Sobald der Bediener das Bedienelement in einer Zwischenstellung des Dosierorgans loslässt, wird das Dosierorgan von der Feder aus der Zwischenstellung in eine Injektionsstellung oder die Nullstellung zurückgestellt. Die Injektionsstellung und die Nullstellung sind vorgesehene Stellungen des Dosierorgans. Vorteilhaft wird das Dosierorgan in die nächstniedrige vorgesehene Stellung zurückgestellt, so dass die versehentliche Injektion einer zu hohen Dosis vermieden ist.

Bei üblichen Injektionsgeräten erfolgen das Einstellen der Injektionsdosis und das Injizieren der Injektionsflüssigkeit in unterschiedlichen Bedienbewegungen. Zwischen diesen Bedienbewegungen muss der Bediener das Bedienelement üblicherweise loslassen. Durch die Feder kann auf einfache Weise ein Zurückstellen in die nächstniedrige vorgesehene Stellung des Dosierorgans erreicht werden, wenn der Bediener das Bedienelement nach dem Einstellen der Injektionsdosis und vor dem Auspressen der Injektionsflüssigkeit aus dem Behälter loslässt.

Vorteilhaft wird die Feder beim Einstellen einer Injektionsdosis gespannt. Dabei kann die Feder in Nullstellung des Dosierorgans bereits vorgespannt sein. Es kann jedoch auch vorgesehen sein, dass die Feder in Nullstellung des Dosierorgans weitgehend entspannt ist. Die Feder wirkt zwischen dem Dosierorgan und dem Gehäuse. Vorteilhaft ist die Feder direkt mit dem Dosierorgan und dem Gehäuse verbunden. Dadurch ergibt sich eine günstige Einbausituation. Besonders vorteilhaft ist die Feder eine Schraubenfeder, wobei ein erstes Ende der Feder am Dosierorgan und ein zweites Ende der Feder am Gehäuse eingehängt sind. Es kann jedoch auch vorgesehen sein, dass ein Ende der Feder an einem drehfest im Gehäuse gehaltenen Bauteil festgelegt ist. Dadurch kann sich ein vereinfachter Aufbau der Einzelteile des Injektionsgeräts ergeben.

Die Rasteinrichtung gibt vorteilhaft beim Erreichen einer Injektionsstellung eine für den Bediener hörbare und/oder spürbare Rückmeldung. Vorteilhaft übt die Rasteinrichtung in Zwischenstellungen des Dosierorgans keine Kraft auf das Dosierorgan aus. Die Rückstellung des Dosierorgans in eine Injektionsstellung oder die Nullstellung erfolgt dadurch nicht entgegen einer von der Rasteinrichtung ausgeübten Kraft. Dadurch wird eine einfache und sichere Rückstellung erreicht, da die von der Feder ausgeübte Rückstellkraft nicht von einer von der Rasteinrichtung ausgeübten Kraft überlagert wird. Dadurch, dass die Rasteinrichtung in Zwischenstellungen des Dosierorgans keine Kraft auf das Dosierorgan ausübt, können außerdem auch bei geringem zur Verfügung stehendem Bauraum definierte Rastpositionen erreicht werden. Ein einfacher Aufbau ergibt sich, wenn die Rasteinrichtung mindestens eine federnde Raste aufweist, die mit einem Rastelement zusammenwirkt.

**Das** Dosierorgan **ist** zur Einstellung einer Injektionsdosis um eine Längsmittelachse des Injektionsgeräts drehbar. Dadurch ergeben sich ein kompakter Aufbau und eine einfache Handhabung.

Bei einem drehbaren Dosierorgan ist vorgesehen, dass die Injektionsstellungen in Umfangsrichtung um die Längsmittelachse einen Winkelabstand von mindestens etwa 30° zueinander aufweisen. Bei Winkelabständen von etwa 30° oder mehr kann allein aufgrund der geometrischen Gestaltung der Rasteinrichtung eine Rückstellung in vorgesehene Stellungen des Dosierorgans nicht mehr sicher gewährleistet werden. Der Winkelabstand zwischen zwei Injektionsstellungen beträgt vorteilhaft mindestens etwa 45°, insbesondere mindestens etwa 60°. Der Winkelabstand ist vorteilhaft so gewählt, dass ein ganzzahliges Vielfaches des Winkelabstands 360° ergibt.

Vorteilhaft wirkt die Rasteinrichtung zwischen einem Zustellteil und dem Gehäuse, wobei das Zustellteil zur Einstellung einer Injektionsdosis um die Längsmittelachse des Injektionsgeräts drehbar ist. Vorteilhaft ist das Zustellteil beim Einstellen einer Injektionsdosis drehfest mit dem Dosierorgan verbunden. Beim Auspressen der Dosis bewegt sich das Zustellteil vorteilhaft in Richtung der Längsmittelachse gegenüber dem Gehäuse. Dadurch wird erreicht, dass beim Auspressen der Injektionsflüssigkeit die Rasteinrichtung nicht wirkt und keine Rastschritte für den Bediener hörbar oder spürbar sind. Die Rasteinrichtung kann außerdem so ausgebildet sein, dass ein Zurückdrehen des Zustellteils nach Erreichen einer Injektionsstellung in die nächstniedrige Injektionsstellung nicht mehr möglich ist. Ein einfacher Aufbau ergibt sich, wenn das Zustellteil an mindestens einem Längssteg des Gehäuses geführt ist. Vorteilhaft ist an dem Längssteg mindestens ein Rastelement der Rasteinrichtung ausgebildet. Dadurch ergibt sich ein einfacher Aufbau. Der Längssteg bildet gleichzeitig ein Rastelement und die Längsführung für das Zustellteil.

Das Bedienelement ist mehrteilig ausgebildet und umfasst einen Betätigungsknopf und die Stellhülse. Die Stellhülse ist **insbesondere** fest mit dem Dosierorgan verbunden. Der Betätigungsknopf ist vorteilhaft über einen Mitnehmer mit dem Zustellteil verbunden, wobei der Betätigungsknopf zum Auspressen von Injektionsflüssigkeit aus dem Behälter in Richtung der Längsmittelachse in proximaler Richtung des Injektionsgeräts verschoben wird. Dadurch ergibt sich eine einfache, intuitive Bedienung des Injektionsgeräts. Die "proximale Richtung" bezeichnet dabei die Injektionsrichtung, also in Richtung zu einer Aufnahme für die Injektionsnadel hin bzw. die Richtung, in der die Injektionsflüssigkeit aus dem Behälter ausgepresst wird. Die "distale Richtung" bezeichnet die entgegengesetzte Richtung, also von der Injektionsnadel weg. Das distale Ende des Injektionsgeräts ist das der Injektionsnadel abgewandt liegende Ende. Mit "proximal" wird die Seite des Injektionsgeräts bezeichnet, die bei einer Injektion der Einstichstelle zugewandt liegt, und mit "distal" die Seite, die der Einstichstelle abgewandt liegt. Eine einfache Gestaltung des Injektionsgeräts ergibt sich, wenn der Betätigungsknopf einteilig mit dem Mitnehmer ausgebildet ist. Es kann jedoch auch vorgesehen sein, dass der Mitnehmer mit dem Betätigungsknopf axial fest, aber gegenüber dem Betätigungsknopf drehbar verbunden ist.

Der Betätigungsknopf ist vorteilhaft über eine Kupplung mit der Stellhülse verbunden, die in einer ersten, distalen Position des Betätigungsknopfs eine drehfeste Verbindung zwischen dem Mitnehmer und der Stellhülse herstellt und in einer zweiten, proximalen Position des Betätigungsknopf eines Drehung der Stellhülse gegenüber dem Mitnehmer zulässt. Dadurch kann erreicht werden, dass das Zustellteil beim Einstellen der Injektionsdosis mit dem Dosierorgan mitdreht und beim Auspressen der Injektionsflüssigkeit aus dem Behälter in Längsrichtung des Injektionsgeräts geführt und gegenüber dem Gehäuse nicht drehbar ist, während sich das Dosierorgan um die Längsmittelachse des Injektionsgeräts dreht. Die Drehbewegung des Zustellteils bewirkt vorteilhaft beim Einstellen der Injektionsdosis über eine erste Gewindeverbindung eine axiale Bewegung des Zustellteils um einen ersten Stellweg in Richtung der Längsmittelachse des Injektionsgeräts. Das Zustellteil wird dabei vorteilhaft in distaler Richtung verschoben.

Um eine genaue Einstellung einer Injektionsdosis zu erlauben, ist vorgesehen, dass das Dosierorgan sich beim Einstellen einer Injektionsdosis in Richtung der Längsmittelachse des Injektionsgeräts, vorzugsweise in distaler Richtung, bewegt und dass die Bewegungen von Zustellteil und Dosierorgan in Richtung der Längsmittelachse des Injektionsgeräts sich voneinander unterscheiden. Das Dosierorgan ist über eine zweite Gewindeverbindung mit dem Gehäuse verbunden, die die Drehbewegung des Dosierorgans in eine Bewegung des Dosierorgans und des Bedienelements in Richtung der Längsmittelachse des Injektionsgeräts um einen zweiten Stellweg bewirkt. Der zweite Stellweg, um den sich das Dosierorgan bewegt, ist dabei insbesondere größer als der erste Stellweg, um den sich das Zustellteil bewegt. Auch das Dosierorgan bewegt sich dabei vorteilhaft in distaler Richtung. Vorteilhaft besitzt das Injektionsgerät einen Schieber, der ein Gewinde einer dritten Gewindeverbindung trägt. Die Drehbewegung des Schiebers bewirkt dabei eine Bewegung in distaler Richtung der Längsmittelachse um einen dritten Stellweg. Der dritte Stellweg, um den sich der Schieber bewegt, ist vorteilhaft mindestens so groß wie der erste Stellweg, um den sich das Zustellteil bewegt. Der Schieber besitzt insbesondere einen Mitnahmeabsatz, der mit einem Mitnahmeabsatz des Zustellteils zusammenwirkt. Der Schieber kann dadurch beim Auspressen von Injektionsflüssigkeit auf das Zustellteil wirken und dieses in proximaler Richtung verschieben.

Ein Ausführungsbeispiel der Erfindung wird im Folgenden anhand der Zeichnung erläutert. Es zeigen:
- Fig. 1: eine Seitenansicht eines Ausführungsbeispiels eines Injektionsgeräts,
- Fig. 2: einen Schnitt entlang der Linie II-II in Fig. 1,
- Fig. 3: einen Abschnitt des Injektionsgerät aus Fig. 1 nach dem Einstellen einer nicht vorgesehenen Menge an Injektionsflüssigkeit,
- Fig. 4: das Injektionsgerät aus Fig. 1 nach dem Einstellen einer vorgesehenen auszupressenden Menge an Injektionsflüssigkeit,
- Fig. 5: einen Schnitt entlang der Linie V-V in Fig. 4,
- Fig. 6: eine vergrößerte Darstellung des distalen Gehäuseteils des Injektionsgeräts aus Fig. 2,
- Fig. 7: eine ausschnittsweise Schnittdarstellung des Injektionsgeräts aus Fig. 5 im Bereich des Bedienelements nach dem Drücken des Betätigungsknopfs,
- Fig. 8: eine Seitenansicht des Injektionsgeräts bei demontiertem Gehäuse,
- Fig. 9: eine Seitenansicht des Mitnehmers des Injektionsgeräts,
- Fig. 10: einen Schnitt entlang der Linie X-X in Fig. 9,
- Fig. 11: einen Schnitt entlang der Linie XI-XI in Fig. 9,
- Fig. 12: eine Seitenansicht des Dosierorgans des Injektionsgeräts,
- Fig. 13: einen Schnitt entlang der Linie XIII-XIII in Fig. 12,
- Fig. 14: eine Seitenansicht des Dosierorgans in Richtung des Pfeils XIV in Fig. 12,
- Fig. 15: eine Seitenansicht eines Innenrohrs des Injektionsgeräts,
- Fig. 16: einen Schnitt entlang der Linie XVI-XVI in Fig. 15,
- Fig. 17: eine vergrößerte Darstellung der Einzelheit XVII in Fig. 16,
- Fig. 18: eine Seitenansicht des Schiebers des Injektionsgeräts,
- Fig. 19: einen Schnitt entlang der Linie XIX-XIX in Fig. 18,
- Fig. 20: einen Schnitt entlang der Linie XX-XX in Fig. 18,
- Fig. 21: eine Seitenansicht des Zustellteils des Injektionsgeräts,
- Fig. 22: einen Schnitt entlang der Linie XXII-XXII in Fig. 21,
- Fig. 23: einen Schnitt entlang der Linie XXIII-XXIII in Fig. 21,
- Fig. 24: eine Seitenansicht des Zustellteils in Richtung des Pfeils XXIV in Fig. 21,
- Fig. 25: eine Seitenansicht der Kolbenstange des Dosierkolbens des Injektionsgeräts,
- Fig. 26: eine Seitenansicht in Richtung des Pfeils XXVI in Fig. 25,
- Fig. 27: einen Schnitt durch das Innenrohr auf der Höhe der Linie XXVII-XXVII in Fig. 15 mit darin angeordnetem Zustellteil in Sperrstellung des Bedienelements,
- Fig. 28: eine Schnittdarstellung entsprechend Fig. 27 mit dem Zustellteil in Injektionsstellung des Bedienelements,

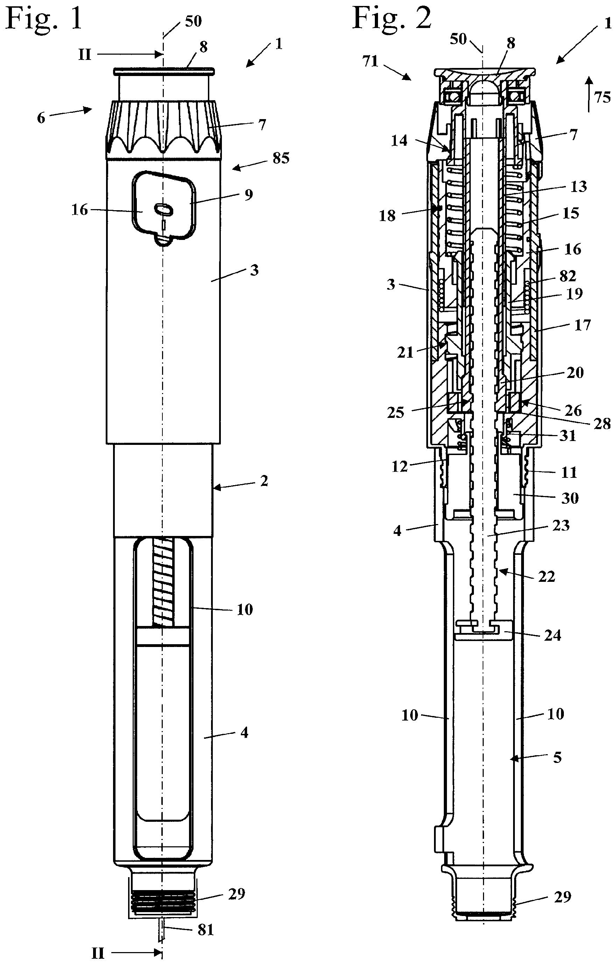

Das in Fig. 1 gezeigte Injektionsgerät 1 besitzt ein Gehäuse 2, das ein oberes, distales Gehäuseteil 3 und einen an der proximalen Seite des oberen Gehäuseteils 3 angeordneten Halter 4 umfasst. An seinem proximalen Ende besitzt der Halter 4 ein Außengewinde 29, an dem eine in Fig. 1 schematisch gezeigte Injektionsnadel 81 aufgeschraubt werden kann. Im Halter 4 ist eine in Fig. 2 gezeigte Aufnahme 5 für einen Behälter mit Injektionsflüssigkeit ausgebildet. Der Behälter mit Injektionsflüssigkeit ist in den Figuren zum ersten Ausführungsbeispiel nicht gezeigt. Wie Fig. 1 zeigt, weist der Halter 4 mindestens eine Aussparung 10 auf, durch die der Behälter mit Injektionsflüssigkeit sichtbar ist. Dadurch kann der Bediener leicht erkennen, ob noch Injektionsflüssigkeit im Behälter vorhanden ist. Wie Fig. 2 zeigt, sind zwei einander gegenüberliegend angeordnete Aussparungen 10 am Halter 4 vorgesehen.

Wie Fig. 1 zeigt, ist am distalen Ende des Gehäuseteils 3 ein Bedienelement 6 angeordnet, das eine Stellhülse 7 und einen an der distalen Seite der Stellhülse 7 angeordneten Betätigungsknopf 8 besitzt. Benachbart zur Stellhülse 7 weist das Gehäuseteil 3 ein Sichtfenster 9 auf, durch das eine auf einem Dosierorgan 16 aufgebrachte Skala erkennbar ist. Das Dosierorgan 16 ist im Gehäuseteil 3 angeordnet. In Fig. 1 zeigt die Skala eine "0", die dem Bediener signalisiert, dass keine Mengeneinstellung vorgenommen wurde. Das Dosierorgan 16 befindet sich in einer Nullstellung 85, in der keine Dosis eingestellt ist.

Fig. 2 zeigt den Aufbau des Injektionsgeräts 1 im Einzelnen, Das Injektionsgerät 1 besitzt einen Mitnehmer 13, der im Wesentlichen hülsenförmig ausgebildet ist und der axial fest mit dem Betätigungsknopf 8 des Bedienelements 6 verbunden ist. Der Begriff "axial" bezieht sich dabei jeweils auf die Richtung einer Längsmittelachse 50 des Injektionsgeräts 1. Der Betätigungsknopf 8 ist mit dem Mitnehmer 13 über eine Schnappverbindung verbunden, die eine Drehung des Betätigungsknopfs 8 gegenüber dem Mitnehmer 13 zulässt. Der Mitnehmer 13 ist über eine Kupplung 14 mit der Stellhülse 7 des Bedienelements 6 verbunden. Bei der in Fig. 2 gezeigten ersten, distalen Position 71 des Betätigungsknopfs 8 ist die Kupplung 14 geschlossen. Die Stellhülse 7 des Bedienelements 6 ist drehfest mit dem Mitnehmer 13 verbunden. Die Stellhülse 7 ist fest mit dem Dosierorgan 16 verbunden, das auch als Einstellglied oder Skalenrohr bezeichnet wird. Der Mitnehmer 13 ist drehfest mit einem Zustellteil 20 verbunden, das über eine erste Gewindeverbindung 25 mit einer Kolbenstange 23 eines Dosierkolbens 22 verbunden ist. Die Kolbenstange 23 trägt an ihrem proximalen Ende eine Kolbenscheibe 24, die zur Anlage an einem Stopfen des Behälters mit Injektionsflüssigkeit dient und über die die Injektionsflüssigkeit aus dem Behälter ausgepresst wird.

Die Kolbenstange 23 ist drehfest in einem Kolbenstangenring 30 gehalten. Der Kolbenstangenring 30 ist axial verschiebbar im Injektionsgerät 1 angeordnet. In der in Fig. 2 gezeigten Stellung, wenn kein Behälter in die Aufnahme 5 eingesetzt ist, wird der Kolbenstangenring 30 von einer Druckfeder 31 in seine proximale Position gedrückt. In dieser Position ist der Kolbenstangenring 30 gegenüber dem Gehäuseteil 3 drehbar. Wird ein Behälter in die Aufnahme 5 eingesetzt und der Halter 4 an einem Befestigungsgewinde 11 mit dem Gehäuseteil 3 verbunden, so drückt der Behälter den Kolbenstangenring 30 in distale Richtung. Das Injektionsgerät 1 besitzt ein Innenrohr 17, das ein Teil des Gehäuses 2 ist und das drehfest und in axialer Richtung fest mit dem Gehäuseteil 3 verbunden ist. An seinem distalen Ende weist der Kolbenstangenring 30 eine Kontur 12 auf, die auf eine Kontur des Innenrohrs 17 abgestimmt ist. In seiner distalen Stellung ist der Kolbenstangenring 30 über die genannten Konturen drehfest mit dem Innenrohr 17 und damit auch drehfest mit dem Gehäuseteil 3 verbunden. Bei in die Aufnahme 5 eingesetztem Behälter ist dadurch die Kolbenstange 23 drehfest im Gehäuseteil 3 gehalten. Durch die drehfeste Verbindung von Kolbenstange 23 und Gehäuseteil 3 bewirkt eine Drehung des Zustellteils 20 eine Bewegung des Zustellteils 20 in distaler Richtung, also in Richtung des Pfeils 75 in Fig. 2. Zwischen dem Zustellteil 20 und dem Innenrohr 17 ist eine Rasteinrichtung 26 gebildet, die Raststellungen des Zustellteils 20 definiert. Bei der in Fig. 2 gezeigten Stellung des Zustellteils 20 liegt das Zustellteil 20 an einem am Innenrohr 17 gebildeten Anschlag 28 an, der die Position des Zustellteils 20 in axialer Richtung definiert.

Das Dosierorgan 16 ist über eine zweite Gewindeverbindung 18 mit dem Innenrohr 17 verbunden. Das Innenrohr 17 ist fest mit dem Gehäuseteil 3 verbunden. Das Innenrohr 17 könnte auch einteilig mit dem Gehäuseteil 3 ausgebildet sein, allerdings wird dadurch die Herstellung des Injektionsgeräts 1 sehr aufwendig. Das Dosierorgan 16 ist drehfest und axial verschiebbar mit einem Schieber 19 verbunden, der ins Innere des Dosierorgans 16 ragt. Der Schieber 19 ist über eine dritte Gewindeverbindung 21 mit dem Innenrohr 17 verbunden. Zwischen dem Mitnehmer 13 und dem Dosierorgan 16 wirkt eine Druckfeder 15, die den Betätigungsknopf 8 in seine erste Position 71 drückt. Zwischen dem Dosierorgan 16 und dem Gehäuse 3 wirkt eine Feder 82. Die Feder 82 ist vorteilhaft als Torsionsfeder ausgebildet. Im Ausführungsbeispiel ist die Feder 82 eine Schraubenzugfeder, die beim Einstellen einer Injektionsdosis sowohl gelängt als auch um die Längsmittelachse 50 des Injektionsgeräts 1 verdreht wird.

Zum Einstellen der auszupressenden Menge an Injektionsflüssigkeit dreht der Bediener das Bedienelement 6, bis die gewünschte Dosis im Sichtfenster 9 erscheint. Dabei dreht sich die Stellhülse 7. Das drehfest mit der Stellhülse 7 verbundene Dosierorgan 16 dreht sich dadurch gegenüber dem oberen Gehäuseteil 3 und dem Innenrohr 17. Aufgrund seiner Drehbewegung wird das Dosierorgan 16 über die zweite Gewindeverbindung 18 in distaler Richtung, also in Richtung des Pfeils 75 verschoben. Das Bedienelement 6 und der axial fest mit dem Betätigungsknopf 8 des Bedienelements 6 verbundene Mitnehmer 13 bewegen sich mit dem Dosierorgan 16. Das Bedienelement 6, der Mitnehmer 13 und das Dosierorgan 16 bewegen sich gemeinsam in distaler Richtung und drehen sich dabei aufgrund der zweiten Gewindeverbindung 18 um die Längsmittelachse 50.

Über die drehfeste Verbindung zwischen Mitnehmer 13 und Zustellteil 20 wird auch das Zustellteil 20 gegenüber dem oberen Gehäuseteil 3 gedreht. Über die erste Gewindeverbindung 25 bewegt sich auch das Zustellteil 20 in distaler Richtung. Der Schieber 19 bewegt sich ebenfalls in distaler Richtung, da der Schieber 19 drehfest mit dem Dosierorgan 16 verbunden ist. Auch der Schieber 19 und das Zustellteil 20 bewegen sich mit einer kombinierten Dreh- und Längsbewegung, wobei der Weg, den Schieber 19 und Zustellteil 20 in Richtung der Längsmittelachse 50 zurücklegen, über die erste Gewindeverbindung 25 bzw. über die dritte Gewindeverbindung 21 festgelegt sind. Es kann auch vorgesehen sein, dass der Schieber 19 über eine dritte Gewindeverbindung mit dem Dosierorgan 16 verbunden ist und drehfest mit dem Gehäuseteil 3 verbunden ist.

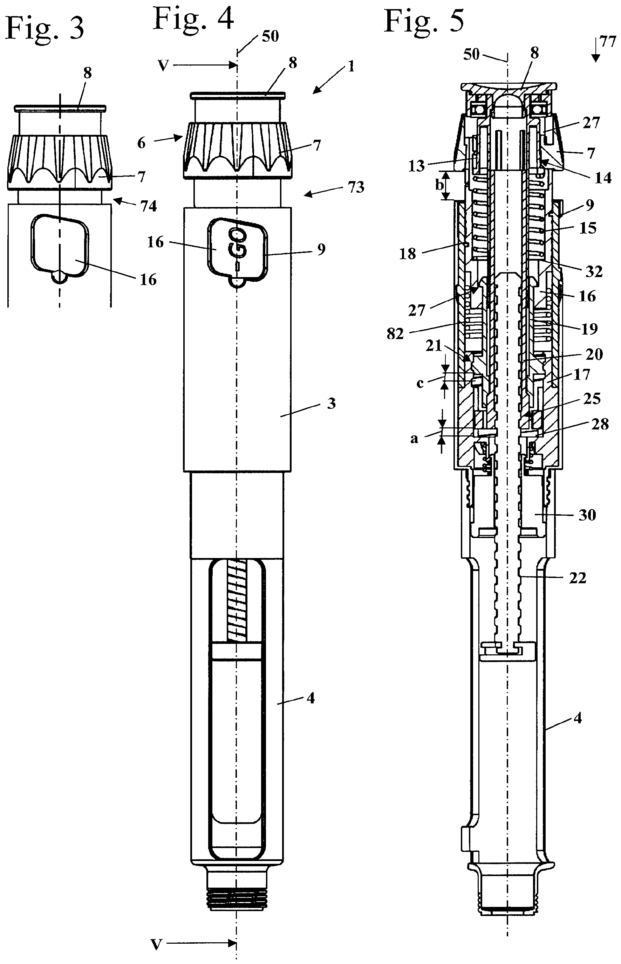

Die Fig. 3 zeigt das Injektionsgerät 1 nach dem Einstellen einer vom Hersteller nicht vorgesehenen Dosis an Injektionsflüssigkeit. Das Dosierorgan 16 befindet sich in einer Zwischenstellung 74, die im Folgenden noch näher erläutert wird.

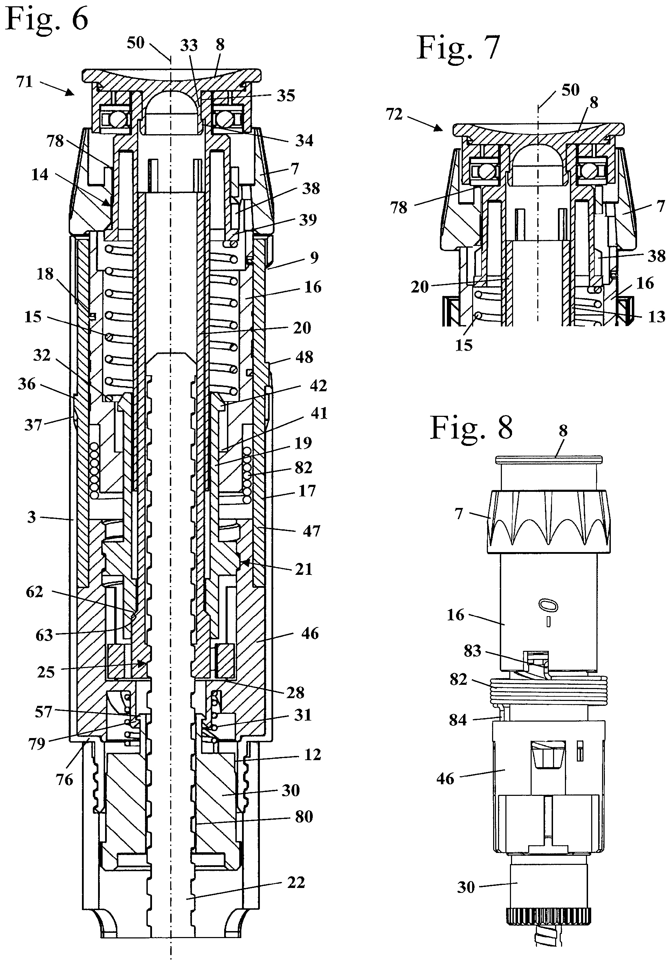

Die Figuren 4 und 5 zeigen das Injektionsgerät 1 nach dem Einstellen einer vorgesehenen auszupressenden Menge an Injektionsflüssigkeit. Das Zustellteil 20 hat sich um einen ersten Stellweg a in distaler Richtung bewegt. Nach dem Einstellen der auszupressenden Menge an Injektionsflüssigkeit hat sich die Stirnseite des Zustellteils 20 um den ersten Stellweg a vom Anschlag 28 entfernt. Das Bedienelement 6 mit der Stellhülse 7 und dem Betätigungsknopf 8 hat sich um einen zweiten Stellweg b in distaler Richtung bewegt. Der zweite Stellweg b ist im Ausführungsbeispiel zwischen der proximalen Stirnseite der Stellhülse 7 und der distalen Stirnseite des Gehäuseteils 3 gemessen. Der zweite Stellweg b ist deutlich größer als der erste Stellweg a. Im Ausführungsbeispiel beträgt der zweite Stellweg b ein Vielfaches, beispielsweise etwa das 3fache des Stellwegs a. Die unterschiedlichen Stellwege a und b ergeben sich durch unterschiedliche Steigungen der ersten Gewindeverbindung 25 und der zweiten Gewindeverbindung 18. Auch das Dosierorgan 16 hat sich um den zweiten Stellweg b in distaler Richtung bewegt. Der Schieber 19 hat sich um einen dritten Stellweg c in distaler Richtung bewegt. Der Stellweg c kann gleich groß sein wie der Stellweg a. Es kann jedoch auch vorgesehen sein, dass der Stellweg c größer als der Stellweg a ist. Der dritte Stellweg c ist in Fig. 5 an der proximalen Stirnseite des Abschnitts des Schiebers 19, der das Gewinde trägt, eingezeichnet, und zwar gegenüber der Position dieser Stirnseite in Fig. 2. Die Feder 82 wurde beim Einstellen der auszupressenden Menge an Injektionsflüssigkeit gespannt. Dabei wurde die Feder 82 um den zweiten Stellweg b gelängt. Gleichzeitig wurden die in Fig. 8 gezeigten Enden 83 und 84 der Feder 82 aufgrund der Drehung des Dosierorgans 16 gegenüber dem Gehäuse 2 um die Längsmittelachse 50 gegeneinander verdreht.

Die maximal einzustellende Menge an Injektionsflüssigkeit ist durch den Weg vorgegeben, den der Betätigungsknopf 6 und das Dosierorgan 16 sich in distaler Richtung bewegen können. Dieser Weg wird durch einen zwischen dem Dosierorgan 16 und dem Schieber 19 gebildeten Anschlag 27 (Fig. 5) begrenzt. Wie Fig. 4 zeigt, besitzt das Dosierorgan 16 an seinem proximalen Ende einen nach innen gerichteten Absatz 41. Dieser Absatz 41 wird von einem Rastrand 42 am Schieber 19 in axialer Richtung hintergriffen. Der Absatz 41 bildet mit dem Rastrand 42 den Anschlag 27. Sobald der Rastrand 42 am Absatz 41 anliegt, ist die maximal einstellbare Menge an Injektionsflüssigkeit erreicht. Der Abstand zwischen Absatz 41 und Rastrand 42 bei dem in den Figuren 1 und 2 gezeigten Zustand des Injektionsgeräts 1 entspricht dem zweiten Stellweg b abzüglich dem ersten Stellweg a.

Wie Fig. 6 zeigt, besitzt das erste Gehäuseteil 3 eine Rastvertiefung 37, die im Ausführungsbeispiel umlaufend ausgebildet ist. In die Rastvertiefung 37 ragt eine am Innenrohr 17 ausgebildete, radial nach außen ragende Raste 36, die das Innenrohr 17 in Richtung der Längsmittelachse 50 des Injektionsgeräts 1 im Gehäuseteil 3 sichert. An seinem proximalen Ende liegt das Innenrohr 17 an einer Schulter 76 des Gehäuseteils 3 an. Zur Drehlagensicherung besitzt das Innenrohr 17 einen nach außen ragenden Zapfen 48, der benachbart zum Sichtfenster 9 am Gehäuseteil 3 einrastet.

Fig. 6 zeigt auch die Abstützung der Druckfeder 15. Die Druckfeder 15 stützt sich mit ihrem proximalen Ende an einer Schulter 32 des Dosierorgans 16 und mit ihrem distalen Ende an einem am Mitnehmer 13 ausgebildeten Rand 39 ab. Der Rand 39 ragt von einem hülsenförmigen Abschnitt des Mitnehmers 13 nach außen. Benachbart zum Rand 39 ist an der distalen Seite des Rands 39 eine Außenverzahnung 38 am Mitnehmer 13 angeordnet. Die Außenverzahnung 38 wirkt mit einer nicht gezeigten Innenverzahnung an der Stellhülse 7 zusammen und bildet mit dieser die Kupplung 14. Bei der in Fig. 6 gezeigten, nicht betätigten Stellung des Betätigungsknopfs 8 ist die Kupplung 14 geschlossen und stellt eine drehfeste Verbindung zwischen Mitnehmer 13 und Stellhülse 7 her. Die Druckfeder 15 drückt den Mitnehmer 13 in Richtung auf die geschlossene Stellung der Kupplung 14. Dadurch wird der Betätigungsknopf 8 in Richtung auf seine distale Position 71 gedrückt.

Nach dem Einstellen der zu injizierenden Dosis kann eine Injektion ausgelöst werden. Hierzu wird der Betätigungsknopf 8 in Richtung des Pfeils 77 in Fig. 5, also in proximaler Richtung, gedrückt. Dadurch bewegt sich der Betätigungsknopf 8 entgegen der Kraft der Druckfeder 15 in Richtung der Längsmittelachse 50 in die Stellhülse 7 hinein, bis der Betätigungsknopf 8 an einem Anschlag 78 der Stellhülse 7 anliegt. Fig. 7 zeigt den Betätigungsknopf 8 in seiner zweiten, proximalen Position 72. In dieser Position hat sich die Außenverzahnung 38 des Mitnehmers 13 aus dem Bereich der Stellhülse 7 bewegt. Dadurch ist die Stellhülse 7 gegenüber Mitnehmer 13 und dem Betätigungsknopf 8 drehbar. Die Kupplung 14 ist offen. Bei weiterem Drücken des Betätigungsknopfs 8 in Richtung des Pfeils 77 in Fig. 5 wird das Dosierorgan 16 in das Innenrohr 17 gedrückt und verschiebt sich dabei in proximaler Richtung. Dabei dreht sich das Dosierorgan 16 aufgrund der zweiten Gewindeverbindung 18. Aufgrund der Drehung des Dosierorgans 16 wird auch der Schieber 19 gedreht und bewegt sich dadurch in proximaler Richtung. Die Drehung des Dosierorgans 16 gegenüber dem Gehäuse 3 wird von der Feder 82 unterstützt. Der Schieber 19 besitzt einen Mitnahmeabsatz 62, der an einem Mitnahmeabsatz 63 des Zustellteils 20 anliegt. Über die Mitnahmeabsätze 62 und 63 drückt der Schieber 19 bei seiner Bewegung in proximaler Richtung auf das Zustellteil 20 und bewegt dieses ebenfalls in proximaler Richtung. Das Zustellteil 20 ist drehfest mit dem Mitnehmer 13 verbunden, der mit dem nicht rotierenden Betätigungsknopf 8 axial fest verbunden ist. Eine Drehung des Zustellteils 20 wird von der Rasteinrichtung 26 verhindert, die beim Einstellen der Dosis in eine Raststellung gestellt worden ist. Der rotierende Schieber 19 kann das Zustellteil 20 dadurch nicht mitdrehen. Da das Zustellteil 20 nicht rotiert und auch der Dosierkolben 22 über den Kolbenstangenring 30 drehfest mit dem Gehäuseteil 3 verbunden ist, sind das Zustellteil 20 und der Dosierkolben 22 fest miteinander verbunden und bewegen sich gemeinsam in proximaler Richtung, bis das Zustellteil 20 am Anschlag 28 anliegt und die eingestellte Menge an Injektionsflüssigkeit vollständig aus dem Behälter ausgepresst wurde.

Das Injektionsgerät 1 ist zur Injektion vorgegebener Dosen von Injektionsflüssigkeit vorgesehen. Das Dosierorgan 16 besitzt mindestens eine in Fig. 4 gezeigte Injektionsstellung 73, in der eine konstruktiv vorgegebene, vorgesehene Menge an Injektionsflüssigkeit eingestellt ist. In Injektionsstellungen 73 rastet die Rasteinrichtung 26 ein. Das Dosierorgan 16 kann auch in mindestens eine Zwischenstellung 74 gestellt werden, die in Fig. 3 gezeigt ist. In einer Zwischenstellung 74 des Dosierorgans 16 ist eine nicht vorgesehene Menge an Injektionsflüssigkeit eingestellt. In Zwischenstellungen 74 des Dosierorgans 16 rastet die Rasteinrichtung 26 nicht ein. Sind nicht vorgesehene Dosen von Injektionsflüssigkeit eingestellt, so wird das Dosierorgan 16 von der Feder 82 in die nächstniedrige Injektionsstellung 73 oder die Nullstellung 85 zurückgestellt, sobald der Bediener die Stellhülse 7 loslässt.

Wie Fig. 6 zeigt, ist das Innenrohr 17 des Gehäuses 2 aus einem proximalen Teil 46 und einem distalen Teil 47 aufgebaut, die fest miteinander verbunden sind. Das Innenrohr 17 kann auch einteilig hergestellt werden. Allerdings ergibt sich dadurch eine deutlich aufwendigere Herstellung des Innenrohrs 17. Um die Herstellung weiter zu vereinfachen, kann es vorteilhaft sein, das Innenrohr 17 aus mehr als zwei Einzelteilen auszubilden. Wie Fig. 8 zeigt, wirkt die Feder 82 zwischen dem distalen Teil 46 des Innenrohrs 17 und dem Dosierorgan 16. Dabei ist ein erstes Ende 83 der Feder 82 am Dosierorgan 16 festgelegt und ein zweites Ende 84 am proximalen Teil 46 des Innenrohrs 17. Die Enden 83 und 84 sind dabei vorteilhaft in entsprechenden Aussparungen von Dosierorgan 16 und proximalem Teil 46 eingehängt.

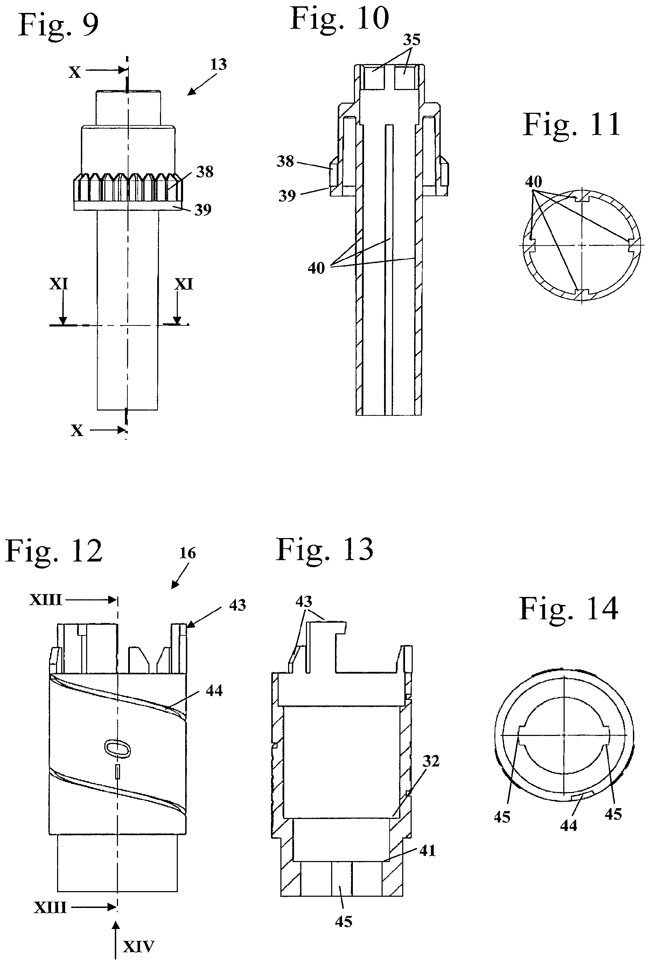

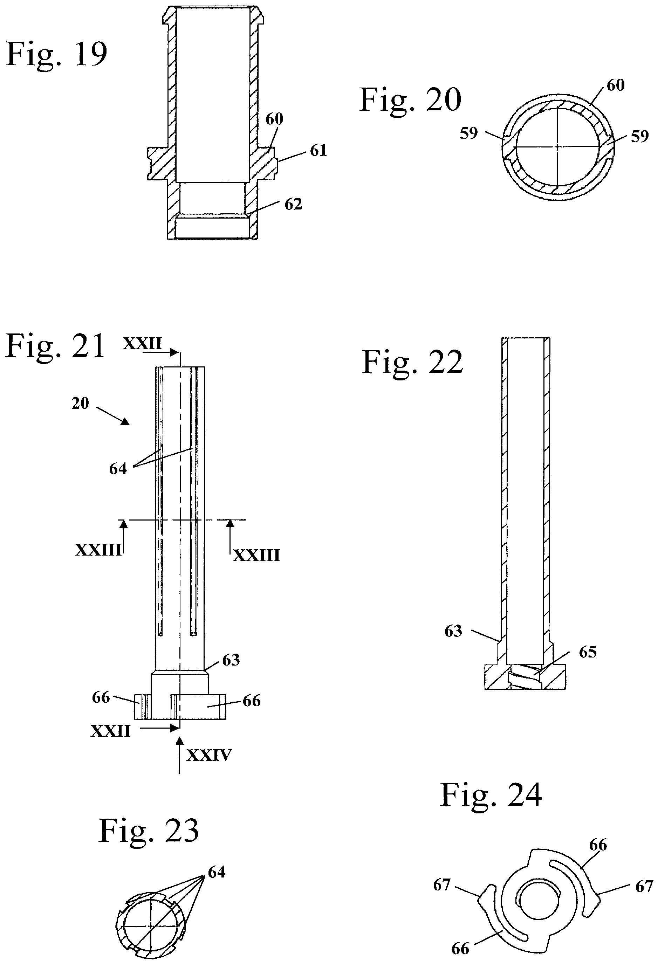

Die Figuren 9 bis 27 zeigen die Bauteile des Injektionsgeräts 1 im Einzelnen. In den Figuren 9 bis 11 ist der Mitnehmer 13 gezeigt. Zur Verbindung mit dem Betätigungsknopf 8 besitzt der Mitnehmer 13 an seinem distalen Ende innenliegende Rasterhöhungen 35, die einen an einem Stutzen 33 des Betätigungsknopfs 8 gebildeten, in Fig. 6 gezeigten Rastrand 34 hintergreifen und dadurch den Betätigungsknopf 8 in axialer Richtung mit dem Mitnehmer 13 verbinden. Der hülsenförmige Mitnehmer 13 besitzt an seinem Innenumfang im Ausführungsbeispiel vier in axialer Richtung verlaufende Führungsstege 40. Die Führungsstege 40 sind auf in Fig. 22 gezeigte Längsnuten 64 des Zustellteils 20 angepasst und greifen in diese ein. Die Führungsstege 40 stellen mit den Längsnuten 64 die drehfeste Verbindung zwischen Mitnehmer 13 und Zustellteil 20 her. Die Führungsstege 40 sind in Richtung der Längsmittelachse 50 des Injektionsgeräts 1 in den Längsnuten 64 frei verschiebbar.

Die Figuren 12 bis 14 zeigen das Dosierorgan 16, das auch als Skalenrohr oder Einstellglied bezeichnet wird. Das Dosierorgan 16 ist hülsenförmig ausgebildet und besitzt an seinem Außenumfang ein Außengewinde 44. Das Außengewinde 44 ist als wendelförmig am Außenumfang des Dosierorgans 16 verlaufende Nut ausgebildet. An seinem distalen Ende trägt das Dosierorgan 16 eine Verbindungskontur 43, die aus haken- und rampenförmigen Elementen gebildet ist, die eine drehfeste Verbindung mit der Stellhülse 7 herstellen. Wie die Figuren 13 und 14 zeigen, besitzt das Dosierorgan 16 an seinem proximalen Ende zwei Führungsnuten 45, die parallel zur Längsmittelachse 50 verlaufen. Die Führungsnuten 45 sind einander gegenüberliegend angeordnet und wirken mit Längsstegen 59 des Schiebers 19 zusammen, die in den Figuren 19 und 21 gezeigt sind. Über die Längsstege 59, die in den Führungsnuten 45 geführt sind, ergibt sich eine drehfeste Verbindung zwischen Dosierorgan 16 und Schieber 19. Die Längsstege 59 sind in Richtung der Längsmittelachse 50 frei in den Führungsnuten 45 beweglich, so dass der Schieber 19 gegenüber dem Dosierorgan 16 in Richtung der Längsmittelachse 50 verschiebbar ist.

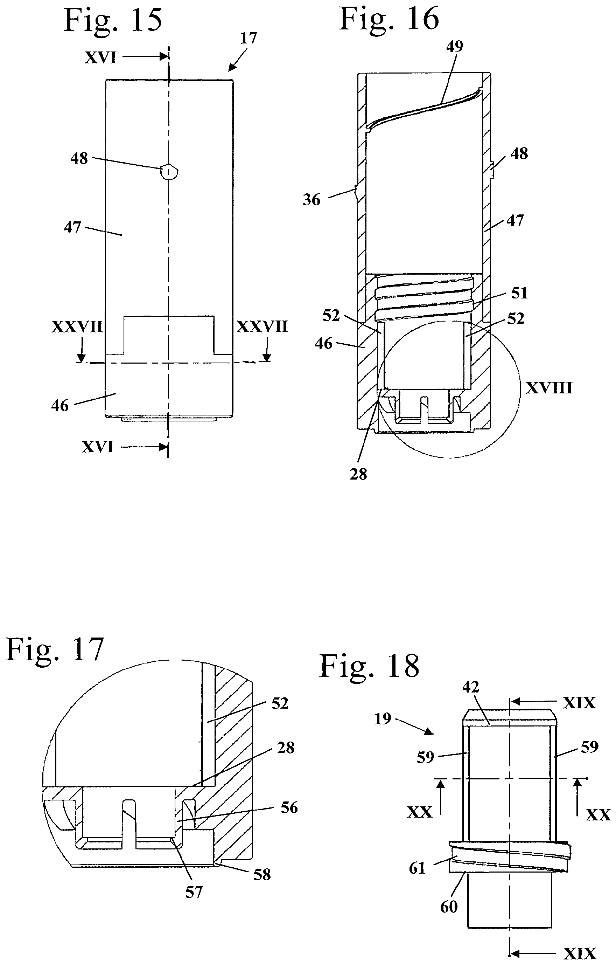

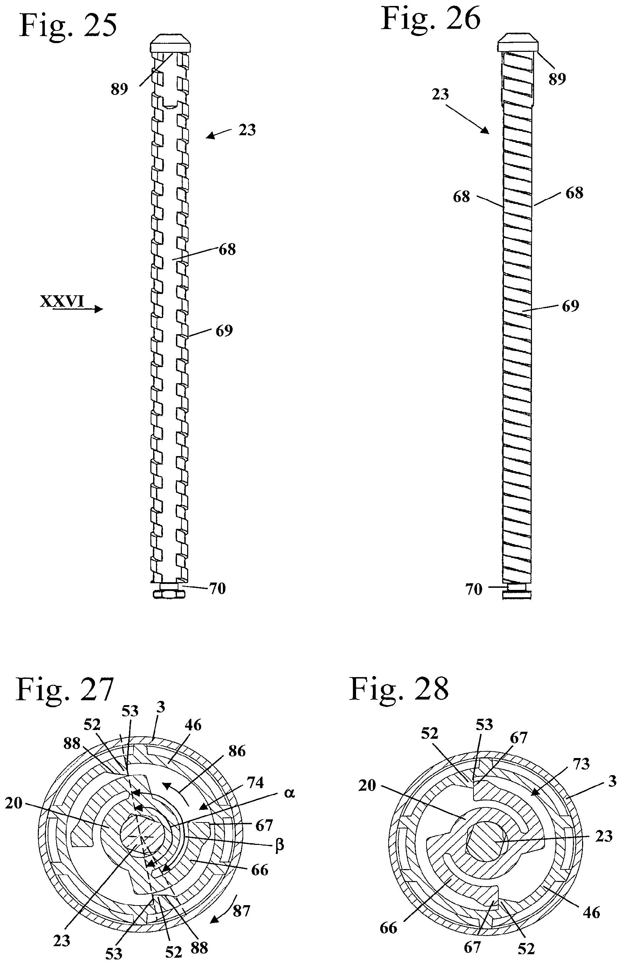

In den Figuren 15 bis 17 ist das Innenrohr 17 gezeigt. Das Innenrohr 17 ist zweiteilig ausgeführt und besteht aus dem proximalen Teil 46 und dem distalen Teil 47, die fest miteinander verbunden sind. Im distalen Teil 47 des Innenrohrs 17 ist ein Innengewinde 49 angeordnet, das aus einem spiralförmig am Innenumfang verlaufenden Steg gebildet ist. Das Innengewinde 49 ist durch einen einzigen Gewindegang gebildet. Es kann vorgesehen sein, das Innengewinde 49 nur durch einen oder mehrere Teilabschnitte eines Gewindegangs auszubilden. Das Innengewinde 49 wirkt mit dem Außengewinde 44 des Dosierorgans 16 zusammen und bewirkt eine axiale Verschiebung des Dosierorgans 16 bei einer Drehung des Dosierorgans 16. Im proximalen Teil 46 des Innenrohrs 17 ist ein Innengewinde 51 ausgebildet, das mit einem in Fig. 18 gezeigten Außengewinde 61 des Schiebers 19 zusammenwirkt. Das Innengewinde 51 bildet mit dem Außengewinde 61 die dritte Gewindeverbindung 21. An der proximalen Seite des Innengewindes 51 schließen sich Längsstege 52 an. Wie Fig. 27 zeigt, sind im Ausführungsbeispiel insgesamt zwei Längsstege 52 vorgesehen, die etwa einander gegenüberliegend angeordnet sind. Die Längsstege 52 definieren mit den beiden einander gegenüberliegenden Rasten 67 des Zustellteils 20 die Raststellungen der Rasteinrichtung und damit die Injektionsstellungen 73. Die beiden Injektionsstellungen 73 besitzen im Ausführungsbeispiel einen Winkelabstand α von 180°. Der Winkelabstand α beträgt vorteilhaft mindestens etwa 30°, insbesondere mindestens etwa 45°, besonders vorteilhaft mindestens 60°. Die Längsstege 52 besitzen in Umfangsrichtung einen in Fig. 27 gezeigten Winkelabstand β zueinander, der im Ausführungsbeispiel etwas weniger als 180°, beispielsweise etwa 160° bis 175°, beträgt. Der Winkelabstand β entspricht dem Winkelbereich, in dem das Dosierorgan 16 zwischen zwei Injektionsstellungen 73 oder einer Injektionsstellung 73 und einer Nullstellung 85 in Zwischenstellungen 74 gestellt werden kann. Vorteilhaft übt in diesem Winkelbereich die Rasteinrichtung 26 keine Kraft auf das Zustellteil 20 und damit auf das Dosierorgan 16 aus. Es kann auch eine andere Anzahl von Längsstegen 52 und/oder Rasten 67 vorteilhaft sein. Beispielsweise können vier Längsstege 52 und zwei Rasten 67 vorgesehen sein, die so angeordnet sind, dass sich ein Winkelabstand α von 90° zwischen den Injektionsstellungen 73 ergibt.

Im Ausführungsbeispiel sind die Längsstege 52 und die Rasten 67 so gestaltet, dass ein Zurückdrehen des Bedienelements 6 aus einer Raststellung in eine Stellung, die einer geringeren Menge an Injektionsflüssigkeit zugeordnet ist, nicht möglich ist. Es kann jedoch auch vorgesehen sein, dass die Form der Längsstege 52 und Rasten 67 ein Zurückdrehen des Bedienelements 6 erlauben, beispielsweise durch in Umfangsrichtung symmetrische Gestaltung.

Wie Fig. 17 zeigt, ragt am proximalen Ende des Innenrohrs 17 ein Zentrierrand 58 in proximale Richtung. Der Zentrierrand 58 ragt in eine proximale Öffnung des Gehäuseteils 3 und stellt einen festen Sitz des Innenrohrs 17 im Gehäuseteil 3 sicher. An der proximalen Seite des Innenrohrs 17 ragen außerdem Haltestutzen 56 in proximale Richtung, an deren proximalen Ende radial nach innen ragende Rastränder 57 angeformt sind. Die Rastränder 57 wirken mit einem Rastrand 79 des Kolbenstangenrings 30 zusammen, der in Fig. 5 gezeigt ist. Der Rastrand 79 stellt mit dem Rastrand 57 eine axiale Sicherung für den Kolbenstangenring 30 dar. Wie Fig. 5 zeigt, drückt die zweite Druckfeder 31 den Kolbenstangenring 30 in seine proximale Position, bis der Rastrand 79 am Rastrand 57 anliegt. In dieser Position kann der Bediener das Gehäuseteil 3 gegenüber dem Kolbenstangenring 30 drehen, um den Dosierkolben 22 in distale Richtung zu bewegen. Dies ist beim Auswechseln eines Behälters für Injektionsflüssigkeit vorgesehen.

In den Figuren 18 bis 20 ist der Schieber 19 im Einzelnen gezeigt. An seinem distalen Ende besitzt der Schieber 19 den Rastrand 42. Wie die Figuren 18 und 19 zeigen, ist das Außengewinde 61 an einem Ringsteg 60 ausgebildet, der radial nach außen ragt. Auch der Schieber 19 ist im Wesentlichen hülsenförmig ausgebildet.

Die Figuren 21 bis 24 zeigen das Zustellteil 20. An seinem proximalen Ende besitzt das Zustellteil 20 zwei Rastarme 66, die in Fig. 24 gezeigt sind. An ihrem freien Ende besitzen die Rastarme 66 jeweils eine Raste 67, die radial nach außen weist. Die Rastarme 66 verlaufen etwa in Umfangsrichtung und sind radial nach außen federnd ausgebildet. Fig. 22 zeigt ein am proximalen Ende des Zustellteils 20 ausgebildetes Innengewinde 65, das mit dem Dosierkolben 22 zusammenwirkt. Das Innengewinde 65 und die Rastarme 65 sind im gleichen Längsabschnitt des Zustellteils 20 angeordnet.

Wie die Figuren 25 und 26 zeigen, besitzt die Kolbenstange 23 ein Außengewinde 69, das mit dem Innengewinde 65 des Zustellteils 20 zusammenwirkt und mit diesem die erste Gewindeverbindung 25 bildet. An ihren gegenüberliegenden Längsseiten besitzt die Kolbenstange 23 Abflachungen 68, die zur Sicherung der Drehlage der Kolbenstange 23 mit entsprechenden Abflachungen einer in Fig. 5 gezeigten Öffnung 80 im Kolbenstangenring 30 zusammenwirken. An ihrem proximalen Ende besitzt die Kolbenstange 23 eine Befestigungsnut 70, an der die Kolbenscheibe 24 gehalten ist. An ihrem distalen Ende besitzt die Kolbenstange 23 einen Anschlag 89. Am distalen Ende der Kolbenstange 23 endet das Außengewinde 69 in einer Kontur, die im Ausführungsbeispiel einen runden Querschnitt mit einem Durchmesser aufweist, der größer als der Außendurchmesser des Außengewindes 69 ist. Die proximale Fläche dieser Kontur bildet den Anschlag 89 zum Innengewinde 68 des Zustellteils 20. Am Anschlag 89 besitzt die Kolbenstange 23 im Ausführungsbeispiel einen runden Querschnitt, dessen Außendurchmesser etwa dem größten Außendurchmesser der Kolbenstange 23 entspricht. Dadurch kann der Anschlag 89 nicht in das Innengewinde 65 des Zustellteils 20 eingeschraubt werden. Auch eine andere Gestaltung des Anschlags 89, die ein Einschrauben in das Innengewinde 65 verhindert, kann jedoch vorteilhaft sein. Der Anschlag 89 ist so angeordnet, dass der Anschlag 89 am Zustellteil 20 anschlägt, wenn die noch im Behälter vorhandene Menge an Injektionsflüssigkeit eingestellt ist. Dadurch kann der Bediener keine Dosis einstellen, die größer als die noch im Behälter vorhandene Restmenge an Injektionsflüssigkeit ist.

Fig. 27 zeigt die Anordnung des Zustellteils 20 in einer Zwischenstellung 74 von Bedienelement 6, Dosierorgan 16 und Zustellteil 20. Die Rasten 67 sind zu den Längsstegen 52 beabstandet. Die Feder 82 (Fig. 8) wirkt auf das Dosierorgan 16 in Richtung auf die Nullstellung 85 des Dosierorgans 16. Über die beim Einstellen der Injektionsdosis geschlossene Kupplung 14 und den Mitnehmer 13 wirkt die Feder 82 auch auf das Zustellteil 20. Das Zustellteil 20 wird in Richtung des in Fig. 27 gezeigten Pfeils 86 in Richtung auf die vorangegangene Raststellung der Rasten 67 belastet. Sobald der Bediener die Stellhülse 7 loslässt, beispielsweise, um den Bedienknopf 8 zu drücken und eine Dosis zu injizieren, werden das Dosierorgan 16 und das Zustellteil 20 aufgrund der Kraft der Feder 82 bis zur vorangegangenen vorgesehenen Stellung, die der nächstkleineren vorgesehenen Dosis oder keiner Dosis von Injektionsflüssigkeit zugeordnet ist, zurückgestellt. Dabei dreht sich das Zustellteil 20 in Richtung des Pfeils 86. Vorgesehene Stellungen sind dabei Injektionsstellungen 73 oder die Nullstellung 85. Da keine nicht vorgesehene Menge an Injektionsflüssigkeit eingestellt werden kann, ist das Auspressen einer nicht vorgesehenen Menge an Injektionsflüssigkeit verhindert.

Wird das Bedienelement 6 und damit auch das Zustellteil 20 weiter gedreht, so gelangen die Rasten 67 nach Überwinden der Längsstege 52, die mit den Rasten 67 die Rasteinrichtung 26 bilden, in Drehrichtung 87 hinter die Längsstege 52. Die Drehrichtung 87 ist die Drehrichtung, in der sich das Zustellteil 20 und das Dosierorgan 16 beim Einstellen einer Dosis von Injektionsflüssigkeit drehen. Die Längsstege bilden an ihren in Richtung des Pfeils 86 vorne liegenden Seiten Rastelemente 53, an denen die Rasten 67 einrasten. Liegen die Rasten 67 an den Rastelementen 53 der Längsstege 52 an, so ist die Rasteinrichtung 26 eingerastet, und die Anordnung befindet sich in einer vorgesehenen Injektionsstellung 73. Eine weitere selbsttätige Bewegung des Zustellteils 20 in Richtung des Pfeils 86 aufgrund der Kraft der Feder 82 ist durch die Anlage der Rasten 67 an den Rastelementen 53 vermieden. Der Bediener kann den Bedienknopf 8 drücken und die eingestellte Dosis injizieren. Das Auspressen der eingestellten Menge an Injektionsflüssigkeit wird durch die Feder 82 unterstützt. An den Längsstegen 52 sind die Rasten 67 dabei parallel zur Längsmittelachse 50 des Injektionsgeräts 1 geführt. Die Längsstege 52 stellen dadurch sicher, dass das Zustellteil 20 sich beim Injizieren einer Dosis nicht um die Längsmittelachse 50 drehen und dadurch die auszupressende Menge an Injektionsflüssigkeit verringern kann.

Die Längsstege 52 besitzen an ihren in Drehrichtung 87 vorne liegenden Seiten jeweils eine Schräge 88, die die Rasten 67 radial nach innen auslenken und so das Überwinden der Längsstege 52 erleichtern. Die Schrägen 88 üben auf die Rasten 67 und damit auf das Zustellteil 20 und das Dosierorgan 16 eine der Drehrichtung 87 entgegengerichtete Kraft aus. In Stellungen des Dosierorgans 16, in denen die Rasten 67 an den Schrägen 88 anliegen, wird das Zustellteil 20 aufgrund der von der Rasteinrichtung 26 ausgeübten Kraft entgegen der Drehrichtung 87 zurückgestellt, bis die Rasten 67 nicht mehr an den Schrägen 88 anliegen, wenn der Bediener keine entgegengerichtete Kraft auf das Zustellteil 20 ausübt. In dem Winkelabstand β zwischen den Längsstegen 52 besitzen die Rasten 67 zum proximalen Teil 46 des Innenrohrs 17 einen Abstand und sind nicht in Kontakt mit dem proximalen Teil 46. Vorteilhaft verläuft der Innenumfang des proximalen Teils 46 in diesem Bereich in einem Kreisbogen um die Längsmittelachse 50. In diesem Bereich übt die Rasteinrichtung 26 keine Kraft auf das Zustellteil 20 oder das Dosierorgan 16 aus. Die Rückstellung des Zustellteils 20 und des Dosierorgans 16 in eine Injektionsstellung 73 oder die Nullstellung 85 erfolgt in diesem Bereich ausschließlich aufgrund der Kraft der Feder 82. Im Ausführungsbeispiel ist ein Injektionsgerät 1 gezeigt, bei dem nur eine einzige vorgegebene Menge an Injektionsflüssigkeit aus dem Behälter ausgepresst werden kann. Diese Menge ist erreicht, wenn das Bedienelement um 180° gedreht wurde. Es kann jedoch auch vorgesehen sein, dass mehrere Injektionsstellungen 73 möglich sind, die unterschiedlichen Mengen an Injektionsflüssigkeit zugeordnet sind.

## Patentansprüche

1. Injektionsgerät mit einem Gehäuse (2), in dem eine Aufnahme (5) für einen Behälter mit Injektionsflüssigkeit ausgebildet ist, wobei das Injektionsgerät (1) ein Bedienelement (6) zur Einstellung einer Injektionsdosis besitzt, wobei das Bedienelement (6) mehrteilig ausgebildet ist und einen Betätigungsknopf (8) und eine Stellhülse (7) besitzt, wobei das Injektionsgerät (1) ein Innenrohr (17) besitzt, das fest mit einem Gehäuseteil (3) des Gehäuses (2) verbunden ist, wobei das Injektionsgerät (1) ein Dosierorgan (16) besitzt, das beim Einstellen der Injektionsdosis gegenüber dem Gehäuse (2) gedreht wird, wobei das Dosierorgan (16) eine Nullstellung (85) und mindestens eine Injektionsstellung (73) besitzt, wobei in der Nullstellung (85) keine Dosis eingestellt ist, und wobei in jeder Injektionsstellung (73) eine vorgesehene Dosis an Injektionsflüssigkeit eingestellt ist, wobei das Dosierorgan (16) über eine Gewindeverbindung (18) mit dem Innenrohr (17) verbunden ist, wobei die Stellhülse (7) drehfest mit dem Dosierorgan (16) verbunden ist, wobei sich beim Einstellen einer auszupressenden Menge an Injektionsflüssigkeit die Stellhülse (7) dreht und das drehfest mit der Stellhülse (7) verbundene Dosierorgan (16) sich dadurch gegenüber dem Gehäuseteil (3) und dem Innenrohr (17) dreht und das Dosierorgan (16) aufgrund seiner Drehbewegung über die Gewindeverbindung (18) in distaler Richtung verschoben wird, wobei das Injektionsgerät (1) eine Rasteinrichtung (26) besitzt, die zwischen zwei beim Einstellen einer Injektionsdosis relativ zueinander bewegten Teilen wirkt, und wobei jeder Injektionsstellung (73) des Dosierorgans (16) eine Raststellung der Rasteinrichtung (26) zugeordnet ist, **dadurch gekennzeichnet, dass** das Dosierorgan (16) in mindestens eine Zwischenstellung (74) stellbar ist, in der keine vorgesehene Dosis an Injektionsflüssigkeit eingestellt ist, und dass zwischen dem Dosierorgan (16) und dem Gehäuse (2) eine Feder (82) wirkt, die das Dosierorgan (16) bei unbetätigtem Bedienelement (6) aus einer Zwischenstellung (74) in eine Injektionsstellung (73) oder die Nullstellung (85) zurückstellt.

2. Injektionsgerät nach Anspruch 1,
**dadurch gekennzeichnet, dass** die Feder (82) beim Einstellen einer Injektionsdosis gespannt wird.

3. Injektionsgerät nach Anspruch 1 oder 2,
**dadurch gekennzeichnet, dass** die Rasteinrichtung (26) in Zwischenstellungen (74) des Dosierorgans (16) keine Kraft auf das Dosierorgan (16) ausübt.

4. Injektionsgerät nach einem der Ansprüche 1 bis 3,
**dadurch gekennzeichnet, dass** mindestens zwei Injektionsstellungen (73) vorgesehen sind, die in Umfangsrichtung um die Längsmittelachse (50) einen Winkelabstand (β, δ) von mindestens etwa 30° zueinander aufweisen.

5. Injektionsgerät nach einem der Ansprüche 1 bis 4,
**dadurch gekennzeichnet, dass** die Rasteinrichtung (26) zwischen einem Zustellteil (20) und dem Gehäuse (2) wirkt, wobei das Zustellteil (20) zur Einstellung einer Injektionsdosis um die Längsmittelachse (50) des Injektionsgeräts (1) drehbar ist.

6. Injektionsgerät nach Anspruch 5,
**dadurch gekennzeichnet, dass** das Zustellteil (20) beim Einstellen einer Injektionsdosis drehfest mit dem Dosierorgan (16) verbunden ist, und dass das Zustellteil (20) sich beim Auspressen der Dosis in Richtung der Längsmittelachse (50) gegenüber dem Gehäuse (2) bewegt und an mindestens einem Längssteg (52) des Gehäuses (2) geführt ist.

7. Injektionsgerät nach Anspruch 6,
**dadurch gekennzeichnet, dass** an dem Längssteg (52) mindestens ein Rastelement (53) der Rasteinrichtung (26) ausgebildet ist.

8. Injektionsgerät nach einem der Ansprüche 5 bis 7,
**dadurch gekennzeichnet, dass** der Betätigungsknopf (8) über einen Mitnehmer (13) mit dem Zustellteil (20) verbunden ist, und dass der Betätigungsknopf (8) zum Auspressen von Injektionsflüssigkeit aus dem Behälter in Richtung der Längsmittelachse (50) in proximaler Richtung des Injektionsgeräts (1) verschoben wird.

9. Injektionsgerät nach Anspruch 8,
**dadurch gekennzeichnet, dass** der Betätigungsknopf (8) über eine Kupplung (14) mit der Stellhülse (7) verbunden ist, wobei die Kupplung (14) in einer ersten, distalen Position (71) des Betätigungsknopfs (8) eine drehfeste Verbindung zwischen dem Mitnehmer (13) und der Stellhülse (7) herstellt und in einer zweiten, proximalen Position (72) des Betätigungsknopfs (8) eine Drehung der Stellhülse (7) gegenüber dem Mitnehmer (13) zulässt.

10. Injektionsgerät nach einem der Ansprüche 5 bis 9,
**dadurch gekennzeichnet, dass** die Drehbewegung des Zustellteils (20) beim Einstellen der Injektionsdosis über eine erste Gewindeverbindung (25) eine axiale Bewegung des Zustellteils (20) bewirkt, wobei das Zustellteil (20) um einen ersten Stellweg (a) in Richtung der Längsmittelachse (50) des Injektionsgeräts (1) verschoben wird und dass die Gewindeverbindung (18), über die das Dosierorgan (16) mit dem Innenrohe (17) verbunden ist, eine zweite Gewindeverbindung (18) ist.

11. Inj ektionsgerät nach Anspruch 10,
**dadurch gekennzeichnet, dass** die Drehbewegung des Dosierorgans (16) eine Bewegung des Dosierorgans (16) und des Bedienelements (6) in Richtung der Längsmittelachse (50) des Injektionsgeräts (1) um einen zweiten Stellweg (b) bewirkt, wobei der zweite Stellweg (b) größer als der erste Stellweg (a) ist.

12. Injektionsgerät nach Anspruch 11,
**dadurch gekennzeichnet, dass** das Injektionsgerät (1) einen Schieber (19) besitzt, der ein Gewinde einer dritten Gewindeverbindung (21) trägt, wobei die Drehbewegung des Schiebers (19) eine Bewegung in distaler Richtung der Längsmittelachse (50) um einen dritten Stellweg (c) bewirkt, der mindestens so groß wie der erste Stellweg (a) ist, und dass der Schieber (19) einen Mitnahmeabsatz (62) besitzt, der mit einem Mitnahmeabsatz (63) des Zustellteils (20) zusammenwirkt und der eine axiale Bewegung des Schiebers (19) in proximaler Richtung auf das Zustellteil (20) überträgt.

## Claims

1. Injection device having a housing (2), in which a receptacle (5) for a container with injection liquid is formed, wherein the injection device (1) has an operating element (6) for setting an injection dose, wherein the operating element (6) is designed in several parts and has an operating button (8) and a adjusting sleeve (7), wherein the injection device (1) has an inner tube (17) permanently connected to a housing part (3) of the housing (2), wherein the injection device (1) has a metering element (16), which is turned with respect to the housing (2) when setting the injection dose, wherein the metering element (16) has a zero position (85) and at least one injection position (73), wherein no dose is set in the zero position (85) and wherein a provided dose of injection liquid is set in each injection position (73), wherein the metering element (16) is connected to the inner tube (17) via a threaded connection (18), wherein the adjusting sleeve (7) is non-rotatably connected to the metering element (16), wherein, when setting a quantity of injection liquid to be expressed, the adjusting sleeve (7) turns and the metering element (16), being non-rotatably connected to the adjusting sleeve (7), turns with respect to the housing part (3) and the inner tube (17) as a result and the metering element (16) is displaced in the distal direction by way of the threaded connection (18) by virtue of its rotary movement, wherein the injection device (1) has a latching device (26), which acts between two parts moving relative to each other when setting an injection dose, and wherein each injection position (73) of the metering element (16) is assigned a latching position of the latching device (26),
**characterised in that** the metering element (16) can be set to at least one intermediate position (74), in which no provided dose of injection liquid is set, and **in that** a spring (82) acts between the metering element (16) and the housing (2) which spring (82) resets the metering element (16) from an intermediate position (74) into an injection position (73) or the zero position (85) when the operating element (6) is not actuated,

2. Injection device according to claim 1,
**characterised in that** the spring (82) is tensioned when setting the injection dose.

3. Injection device according to claim 1 or 2,
**characterised in that** the latching device (26) does not apply any force to the metering element (16) in intermediate positions (74) of the metering element (16).

4. Injection device according to any of claims 1 to 3,
**characterised in that** at least two injection positions (73) are provided, which have an angular distance (β, δ) of at least approximately 30° relative to each other in the circumferential direction around the longitudinal central axis (50).

5. Injection device according to any of claims 1 to 4,
**characterised in that** the latching device (26) acts between a feed part (20) and the housing (2), wherein the feed part (20) can be turned about the longitudinal central axis (50) of the injection device (1) for setting an injection dose.

6. Injection device according to claim 5,
**characterised in that** the feed part (20) is non-rotatably connected to the metering element (16) when setting an injection dose, and **in that** the feed part (20), when expressing the dose, moves in the direction of the longitudinal central axis (50) with respect to the housing (2) and is guided along at least one longitudinal web (52) of the housing (2).

7. Injection device according to claim 6,
**characterised in that** at least one latching element (53) of the latching device (26) is formed on the longitudinal web (52).

8. Injection device according to any of claims 5 to 7,
**characterised in that** the operating button (8) is connected to the feed part (20) via an entrainer (13), and **in that** the operating button (8) is displaced in the direction of the longitudinal central axis (50) in the proximal direction of the injection device (1) for expressing injection liquid from the container.

9. Injection device according to claim 8,
**characterised in that** that the operating button (8) is connected to the adjusting sleeve (7) via a clutch (14), wherein the clutch (14) establishes in a first, distal position (71) of the operating button (8) a non-rotatable connection between the entrainer (13) and the adjusting sleeve (7) and in a second, proximal position (72) of the operating button (8) permits a rotation of the adjusting sleeve (7) with respect of the entrainer (13).

10. Injection device according to any of claims 5 to 9,
**characterised in that** the rotary movement of the feed part (20) when setting the injection dose causes an axial movement of the feed part (20) via a first threaded connection (25), wherein the feed part (20) is displaced by a first travel range (a) in the direction of the longitudinal central axis (50) of the injection device (1), and **in that** the threaded connection (18) via which the metering element (16) is connected to the inner tube (17) is a second threaded connection (18).

11. Injection device according to claim 10,
**characterised in that** the rotary movement of the metering element (16) causes a movement of the metering element (16) and the operating element (6) in the direction of the longitudinal central axis (50) of the injection device (1) by a second travel range (b), the second travel range (b) being larger than the first travel range (a).

12. Injection device according to claim 11,
**characterised in that** the injection device (1) has a slide element (19) bearing a thread of a third threaded connection (21), wherein the rotary movement of the slide element (19) causes a movement in the distal direction of the longitudinal central axis (50) by a third travel range (c), which is at least as large as the first travel range (a), and **in that** the slide element (19) has an entrainment shoulder (62), which acts together with an entrainment shoulder (63) of the feed part (20) and transmits an axial movement of the slide element (19) in the proximal direction to the feed part (20).

## Revendications

1. Dispositif d'injection avec un boîtier (2) dans lequel est formé un logement (5) pour un récipient avec un liquide d'injection, dans lequel le dispositif d'injection (1) comporte un élément de commande (6) pour régler une dose d'injection, dans lequel l'élément de commande (6) est réalisé en plusieurs parties et comporte une tête d'actionnement (8) et un manchon de réglage (7), dans lequel le dispositif d'injection (1) comporte un tuyau intérieur (17), qui est relié de manière solidaire à une partie de boîtier (3) du boîtier (2), dans lequel le dispositif d'injection (1) comporte un organe de dosage (16) qui, lors du réglage de la dose d'injection, est tourné par rapport au boîtier (2), dans lequel l'organe de dosage (16) présente une position zéro (85) et au moins une position d'injection (73), dans lequel dans la position zéro (85), aucune dose n'est réglée, et dans lequel dans chaque position d'injection (73) une dose prévue de liquide d'injection est réglée, dans lequel l'organe de dosage (16) est relié au tuyau intérieur (17) par l'intermédiaire d'une liaison filetée (18), dans lequel le manchon de réglage (7) est relié fixe en rotation à l'organe de dosage (16), dans lequel lors du réglage d'une quantité à éjecter de liquide d'injection, le manchon de réglage (7) tourne et l'organe de dosage (16) relié fixe en rotation au manchon de réglage (7) se tourne par rapport à la partie de boîtier (3) et au tuyau intérieur (17) et dans lequel l'organe de dosage (16) est déplacé dans un sens distal du fait de son déplacement en rotation par l'intermédiaire de la liaison filetée (18), dans lequel le dispositif d'injection (1) comporte un dispositif d'enclenchement (26) qui agit entre deux parties déplacées l'une par rapport à l'autre lors du réglage d'une dose d'injection, et une position d'enclenchement du dispositif d'enclenchement (26) est associée à chaque position d'injection (73) de l'organe de dosage (16), **caractérisé en ce que** l'organe de dosage (16) est apte à être amené dans au moins une position intermédiaire (74) dans laquelle aucune dose prévue de liquide d'injection n'est réglée, et **en ce qu'**entre l'organe de dosage (16) et le boîtier (2) agit un ressort (82) qui ramène l'organe de dosage (16), quand l'élément de commande (6) est non actionné, d'une position intermédiaire (74) jusqu'à une position d'injection (73) ou à la position zéro (85).

2. Dispositif d'injection selon la revendication 1,
**caractérisé en ce que** le ressort (82) est contraint lors du réglage d'une dose d'injection.

3. Dispositif d'injection selon l'une des revendications 1 ou 2,
**caractérisé en ce que** le dispositif d'enclenchement (26), dans des positions intermédiaires (74) de l'organe de dosage (16), n'exerce pas de force sur l'organe de dosage (16).

4. Dispositif d'injection selon l'une des revendications 1 à 3,
**caractérisé en ce qu'**il est prévu au moins deux positions d'injection (73), qui présentent dans le sens circonférentiel autour de l'axe longitudinal médian (50) un écartement angulaire (β, δ) d'au moins environ 30°.

5. Dispositif d'injection selon l'une des revendications 1 à 4,
**caractérisé en ce que** le dispositif d'enclenchement (26) agit entre un élément de positionnement (20) et le boîtier (2), dans lequel l'élément de positionnement (20), pour régler une dose d'injection, peut tourner autour de l'axe longitudinal médian (50) du dispositif d'injection (1).

6. Dispositif d'injection selon la revendication 5,
**caractérisé en ce que** l'élément de positionnement (20) est relié fixe en rotation à l'organe de dosage (16), lors du réglage d'une dose d'injection, et **en ce que** l'élément positionnement (20) se déplace lors de l'éjection de la dose en direction de l'axe médian longitudinal (50) par rapport au boîtier (2) et est guidé sur au moins une nervure longitudinale (52) du boîtier (2).

7. Dispositif d'injection selon la revendication 6,
**caractérisé en ce qu'**au moins un élément d'enclenchement (53) du dispositif d'enclenchement (26) est réalisé sur la nervure longitudinale (52).

8. Dispositif d'éjection selon l'une des revendications 5 à 7,
**caractérisé en ce que** le bouton d'actionnement (8) est relié par l'intermédiaire d'un organe d'entraînement (13) à l'élément de positionnement (20), et **en ce que** le bouton d'actionnement (8) est déplacé, pour éjecter le liquide d'injection hors du récipient en direction de l'axe longitudinal médian (50) dans le sens proximal du dispositif d'injection (1).

9. Dispositif d'injection selon la revendication 8,
**caractérisé en ce que** le bouton d'actionnement (8) est relié par l'intermédiaire d'un accouplement (14) au manchon de réglage (7), dans lequel l'accouplement (14) réalise dans une première position distale (71) du bouton d'actionnement (8) une liaison fixe en rotation entre l'organe d'entraînement (13) et le manchon de réglage (7), et autorise dans une seconde position proximale (72) du bouton d'actionnement (8) une rotation du manchon de réglage (7) par rapport à l'organe d'entraînement (13).

10. Dispositif d'injection selon l'une des revendications 5 à 9,
**caractérisé en ce que** le mouvement de rotation de l'élément de positionnement (20), lors du réglage de la dose d'injection, provoque par l'intermédiaire d'une première liaison filetée (25) un mouvement axial dudit élément de positionnement (20), dans lequel l'élément de positionnement (20) est déplacé dans le sens de l'axe longitudinal médian (50) du dispositif d'injection (1) sur une première course de réglage (a), et **en ce que** la liaison filetée (18), par l'intermédiaire de laquelle l'organe de dosage (16) est relié au tuyau intérieur (17), est une deuxième liaison filetée (18).

11. Dispositif d'injection selon la revendication 10,
**caractérisé en ce que** le mouvement de rotation de l'organe de dosage (16) provoque un mouvement de l'organe de dosage (16) et de l'élément de commande (6) dans le sens de l'axe longitudinal médian (50) du dispositif d'injection (1) sur une deuxième course de réglage (b), dans lequel ladite deuxième course de réglage (b) est plus grande que la première course (a).

12. Dispositif d'injection selon la revendication 11,
**caractérisé en ce que** le dispositif d'injection (1) comporte un coulisseau (19) qui porte un filetage d'une troisième liaison filetée (21), dans lequel le mouvement de rotation du coulisseau (19) provoque un mouvement dans le sens distal de l'axe longitudinal médian (50) sur une troisième course de réglage (c) qui est au moins aussi grande que la première course de réglage (a), et **en ce que** le coulisseau (19) comporte un épaulement d'entraînement (62) qui coopère avec un épaulement d'entraînement (63) de l'élément de positionnement (20) et qui transmet un mouvement axial du coulisseau (19) dans le sens proximal à l'élément de positionnement (20).
